# EUROPÄISCHE PATENTSCHRIFT

(11) **EP 2 558 017 B1**
(45) Veröffentlichungstag und Bekanntmachung des Hinweises auf die Patenterteilung: **25.04.2018**
(21) Anmeldenummer: 11743025.6
(22) Anmeldetag: 05.07.2011
(51) Int. Cl.: A61B 18/14, A61B 18/12

(54) **ELEKTRODENANORDNUNG**
ELECTRODE ARRANGEMENT
ENSEMBLE D'ÉLECTRODES

(30) Priorität: 06.07.2010 DE 102010026210
(43) Veröffentlichungstag der Anmeldung: 20.02.2013
(73) Patentinhaber: Sarus GmbH & Co. KG, 35037 Marburg (DE)
(72) Erfinder: Seidel, Wolfgang, 82335 Berg (DE)
(74) Vertreter: Manitz Finsterwald Patentanwälte PartmbB
(86) Internationale Anmeldenummer: PCT/EP2011/003341
(87) Internationale Veröffentlichungsnummer: WO 2012/003966

(56) Entgegenhaltungen:
- EP-A1- 2 184 025
- WO-A2-2007/001981
- US-B1- 6 638 273

## Beschreibung

Die Erfindung betrifft eine Elektrodenanordnung zur Koagulation von Körpergewebe und/oder Körpergefäßen nach dem Oberbegriff des Anspruchs 1 (wie in US6638273 B1 offenbart).

In der Hochfrequenzchirurgie wird ein elektrisches Hochfrequenzfeld an eine Elektrode gelegt, um durch einen Koagulationsstrom Gewebe zu erhitzen und/oder zum Schrumpfen zu bringen. Die Hochfrequenzspannung wird gewöhnlich an eine auf einem aufblasbaren Ballon angeordnete Elektrodenanordnung angelegt, die dann über das Körpergewebe zu einem Koagulationsstrom führt.

Aus der US 6 904 303 B2 ist eine Endoskop-Vorrichtung zur Koagulation von Körpergewebe bekannt, die ein durch eine Flüssigkeit aufblasbaren Ballon bestehend aus wärmeleitfähigem Material und innerhalb des Ballons eine Elektrode zum Erhitzen der Flüssigkeit aufweist. Die Wärme der erhitzten Flüssigkeit wird über den Ballon auf das zu behandelnde Körpergewebe übertragen. Bei einer anderen Ausführungsform wird die erhitzte elektrisch leitfähige Flüssigkeit als Leiter für Hochfrequenz Energie durch poröse Öffnungen des Ballons nach außen auf das Gewebe übertragen.

Aus der US 2007/028 7994 A1 ist ein Endoskop zur Koagulation von Körpergewebe bekannt, bei dem am Ende ein durch eine Flüssigkeit aufblasbarer Ballon vorgesehen ist, der außen eine Vielzahl von längs angeordneten Elektroden aufweist, über die Hochfrequenzenergie zur bipolaren Behandlung auf das Körpergewebe übertragen wird.

Das Körpergewebe wird im Kontakt mit den Elektroden homogen erhitzt. Dies führt zu einer flächenhaften Koagulation. Bei nicht homogen verteilten oder bei unregelmäßig angeordneten Elektrodenanordnungen wird die Flüssigkeit im Ballon irregulär und inhomogen erhitzt. Durch Konvektion der Flüssigkeit im Ballon wird bei länger dauernder Erhitzung der obere Anteil des Ballons heißer, sodass eine inhomogene Wärmeverteilung in und um den Ballon herum die Folge ist.

Eine andere bekannte Elektrodenanordnung zur Koagulation von Körperhohlräumen, z.B. einer Gebärmutterkavität, weist starre vorgeformte Elektroden auf. Eine weitere bekannte Elektrodenanordnung wird zur Koagulation von blutenden gastrointestinalen Ulzera verwendet, wobei hier kleine Spitzen Verwendung finden, die über Ballonkathetern angeordnet sind.

Die Platzierung eines Ballons in einem kleinen Körperhohlraum ist schwierig, da dies nicht unter Sicht erfolgen kann. Es treten Blutkoagel um den Ballon herum auf, die nicht reproduzierbare Bedingungen für die Anwendung elektrischer Felder oder für die Hochfrequenzablation (Gewebedenaturierung) schaffen, und zwar im Vergleich zur Platzierung eines definierten Ballons direkt auf das zu behandelnde Gewebe.

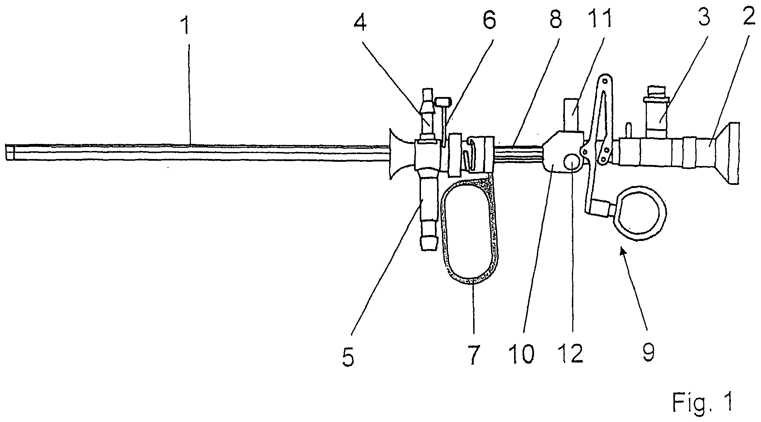

Medizinische Instrumente, insbesondere starre Endoskope zur Resektion von Gewebe sind bekannt. So sind in dem Lehrbuch "Endoskopische Urologie" von Prof. Dr. R. Hofmann, 2. Auflage 2009, Springer Verlag im Kapitel 1 verschiedene endoskopische Resektionsinstrumente beschrieben. Ein solches bekanntes Resektoskop ist in Fig.1 dargestellt. Dieses bekannte Resektoskop besteht aus einem Instrumentenschaft 1, einer Endoskop-Optik 2 mit einem Anschluss für ein Lichtkabel 3 sowie aus einem Arbeitselement. Der Instrumentenschaft 1 weist üblicherweise 24 Charr. oder 26 Charr. Umfang auf. Der Instrumentenschaft 1 kann zur Dauerspülung verwendet werden, d.h. es erfolgt ein separater Spülwasserzufluss 4 sowie eine Absaugung 5 der Irrigationsflüssigkeit (Spülflüssigkeit). Mit einem Hahn 6 wird der Wasserzufluss geregelt. Es kann auch lediglich der Zufluss von Irrigationsflüssigkeit erfolgen, die bei voller Blase durch Entfernen des Arbeitselementes aus dem Instrumentenschaft 1 abgelassen wird.

Das Arbeitselement besteht aus einem Handgriff 7 für den dritten und vierten Finger. Dieser Handgriff 7 ist fest an einem Aufnahmerohr 8 für die Optik 2 und an einem als Drehverschluss ausgebildeten Arretiermechanismus des Arbeitselements mit dem Instrumentenschaft 1 angebracht. Am Handgriff 7 ist ein bewegliches Rückstellelement 9 für den Daumengriff angebracht. Von einem "passiven Resektionsinstrument" spricht man, wenn durch den Federzug eine als Operationselement ausgebildete Operationsschlinge in den Instrumentenschaft 1 eingefahren ist und gegen Federwiderstand ausgefahren wird. Das Arbeitselement kehrt passiv durch Federkraft wieder in den Ausgangszustand zurück. Ein aktives Arbeitselement liegt vor, wenn durch die Federkraft im entspannten Zustand die Operationsschlinge ausgefahren ist und mit dem Daumengriff 9 ein Schlitten 10 gegen die Federkraft eingezogen werden muss.

Am Daumengriff 9 ist der bewegliche Schlitten 10 befestigt, der über das Aufnahmerohr 8 für die Optik 2 gleitet. Meist handelt es sich um ein aus Kunststoff gefertigtes Element, das eine elektrische Zuleitung 11 aufweist sowie einen Knopf 12 zur Arretierung des Elektrodenstabes.

Während einer endoskopischen Operation z.B. bei einer transurethralen Resektion eines gutartigen oder auch bösartigen Prostatagewächses (TURP) kann Gewebe mit einem speziellen Schneidestrom aus einem Hochfrequenz-Spannungsgenerator entweder mit einer bisher gebräuchlichen monopolaren Elektrodenanordnung oder einer bipolaren Elektrodenanordnung reseziert werden. Bei der monopolaren Elektrodenanordnung fließt der Strom von einer aktiven Elektrode durch eine hypotone, wenig leitfähige Lösung (z.B. Glycin-, Mannitol-Lösung) durch den Körper des Patienten zu einer großen flächenhaften Neutralelektrode. Bei der bipolaren Elektrodenanordnung befinden sich zwei Elektroden nahe beieinander. Der Strom fließt durch eine gut leitfähige Lösung, wie z. B. eine Kochsalzlösung von der einen zur anderen Elektrode.

Während einer TURP kommt es in der Regel zu Blutungen. Mit der mit Hochfrequenzspannung versorgten Resektionsschlinge können nun durch einen Koagulationsstrom arterielle und venöse Blutungen gezielt unter Sicht verödet werden. Am Ende der TURP entsteht eine kugelförmige Kavität, auch Loge genannt. Arterielle Blutungen müssen gezielt koaguliert werden. Bei kleinen Arterien wird ein Sistieren einer arteriellen Blutung durch Druck mit einem Ballonkatheter erreicht. Meist liegt bei reduziertem oder abgestelltem Spülstrom am Ende der Operation eine diffuse venöse Blutung aus der gesamten Resektionsfläche vor, die erst durch Einlage eines Ballonkatheters sistiert.

Ein Ballon kann entweder in der Prostata-Loge entfaltet werden und führt dann direkt zur Kompression der venösen Blutung oder er wird z.B. in der benachbart zur Prostata-Loge befindlichen Blase entfaltet und dann zur Prostata-Loge zurückgezogen, so dass durch Kompression der Prostata-Loge eine Blutstillung erreicht wird.

Der transurethrale Katheter (Harnröhrenkatheter) muss gewöhnlich einige Tage zur Vermeidung einer Blasentamponade bespült und belassen werden. Hierdurch entsteht für den Patienten eine erhebliche Morbidität, wie Schmerzen in der Harnröhre und in der Blase. Es treten Blasenkrämpfe, Ausfluss aus der Harnröhre neben dem Katheter, oder Harnwegsinfekte mit Fieber auf. Ein protrahierter Blutverlust aus der großen Wundfläche der Prostata-Loge, bedingt durch das operative Vorgehen, oder eine arterielle oder häufiger eine venöse Blutung, erzeugt durch Reibung und Verletzung der Koagulationsoberfläche der Prostata-Loge durch den Ballon, sind nicht selten.

Bei 2% bis 5% der Operationen nach der TURP entstehen postoperativ Harnröhrenverengungen, wobei die längere Verweildauer einer Einlage eines Katheters in der Harnröhre und eine dadurch bedingte Infektion als Risikofaktoren betrachtet werden. Nicht selten verstopft auch der Katheter mit Blutkoageln, sofern nicht der Dauerspülkatheter regelmäßig bespült wird. Die dabei sich bildenden Blutpfropfen müssen dann aus dem Katheter mit Blasenspritzen ausgespült werden. Gelegentlich muss auch der Katheter selbst ausgetauscht werden oder es muss eine Blasentamponade operativ in Narkose ausgeräumt werden.

Eine ähnliche Situation stellt sich bei der suprapubischen Adenomektomie (Entfernung der Prostata durch die Bauchdecke) dar. Hier wird vom Operateur die große benigne (gutartige) Drüse entweder durch die Prostatakapsel oder transvesikal (durch die Blase) mit dem Finger ausgeschält, so dass eine große Kavität bzw. Loge entsteht. Üblicherweise bleibt eine diffuse venöse Blutung übrig, die durch transurethrale Einlage eines Ballonkatheters, der mit Druck in der Loge entfaltet wird, gestillt wird. Hierdurch entstehen aber dem Patienten für einige Tage bis zur Katheterentfernung erhebliche Beschwerden, wie Schmerzen, Fremdkörpergefühl im Enddarm und in der Harnröhre oder Blase sowie Blasenkrämpfe durch den Fremdkörperreiz.

Daher ist es Aufgabe der Erfindung, eine Elektrodenanordnung der eingangs genannten Art zur Koagulation von Körpergewebe und/oder von Körpergefäßen zu schaffen, bei der zur Vermeidung der genannten Nachteile der bekannten Einrichtungen die Koagulation nach einer Operation einfacher, wirksamer und schneller erfolgen kann.

Diese Aufgabe wird durch die Merkmale des Anspruchs 1 gelöst. Die bandförmigen Elektroden-Paare lassen sich in ihrer entspannten Stellung einfach und schnell an die zu behandelnden Stellen bringen. Auch lassen sie sich in Abhängigkeit von dem zur Verfügung stehenden Raum des Körpergewebes und/oder der Körpergefäße gleichzeitig und schnell in Kontaktberührung mit dem Körpergewebe und/oder den Körpergefäßen aufspannen. Die entsprechend weit aufgespannten Elektrodenpaare liegen dann mit einem gewünschten Druck großflächig an. Eine an allen Elektroden-Paaren angeschaltete Hochfrequenz-Regelspannung des Hochfrequenz-Regelspannungsgenerators generiert dann gleichzeitig und großflächig im Körpergewebe einen bipolaren Koagulationsstrom. Hierdurch wird die Koagulationswirkung verstärkt sowie die Koagulationszeit verkürzt.

Die bandförmigen Elektrodenpaare der Elektrodenanordnung können wie Meridiane eines Ballons aufgespannt werden.

Die Elektrodenanordnung kann im entspannten Zustand durch alle gängigen Resektionsschäfte eingeführt werden und dann unter endoskopischer Sicht entfaltet und in der Prostata-Loge platziert werden. Im aufgespannten Zustand sind die Elektrodenreihen wie Meridiane eines Ballons aufgespannt. Ein Koagulationsstrom fließt jeweils gleichzeitig zwischen den beiden sternförmigen Elektrodenreihen und bewirkt eine flächenhafte bipolare Koagulation, sodass die venöse Blutung oder kleinere arterielle Blutungen gestoppt werden. Durch eine blutungsfreie Loge am Ende der Operation wird die Katheterverweildauer verkürzt. Auf die Einlage eines transurethralen Katheters kann daher verzichtet werden.

Die Koagulation erfolgt mit einem für die Hochfrequenzchirurgie geeigneten Hochfrequenz-Regelspanungsgenerator. Die Regelspannung weist eine Frequenz von etwa 300 bis 600 kHz auf. Die Ausgangsleistung des Hochfrequenz Regelspanungsgenerators beträgt mindestens 300 W. Die Koagulation erfolgt langsam, so dass keine schnelle Karbonisierung des Gewebes erfolgt. Vorteilhaft ist es, wenn sich der Hochfrequenz Regelspanungsgenerator als geregelter Generator an die Impedanz des Systems mit den beiden Elektrodengruppen und dem Körpergewebe anpasst. Vor dem Koagulationsvorgang kann die SWR (Standing Wave Ratio) gemessen und als Ausgangsgröße festgelegt werden. Während des Koagulationsprozesses wird dann die Impedanz ständig gemessen und in einem vorher festgelegten Bereich gehalten. Die Impedanz des Systems liegt in etwa zwischen 10 und 15 Ohm. Die SWR steigt während der Koagulation an. Ein Überschreiten des oberen Grenzwertes führt zur Abschaltung des Systems. Die Operationszeit für den Hochfrequenz Regelspanungsgenerator wird vom Chirurgen festgelegt. Die SWR Kontrolle kann jedoch zu einer vorzeitigen automatischen Abschaltung führen.

Für den suprapubischen (durch Operation von der Bauchdecke aus) Einsatz kann die Anordnung ähnlich verwendet werden, wobei nur ein Handgriff mit Schiebemechanismus zur Faltung und Entfaltung bzw. Aufspannen und Entspannen der Elektrodenanordnung, eine Arretierung für den Elektrodenstab sowie eine Steckverbindung für das Elektrodenkabel notwendig werden.

Weitere vorteilhafte Ausgestaltungen der Erfindung finden sich in den Unteransprüchen.

Wenn eine Operation mittels einer durch einen Instrumentenschaft eingebrachten Operationselementes, z.B. einer Resektionsschlinge, eines Endoskops durchgeführt wird, wird dieses Operationselement nach der Operation entfernt und an seiner Stelle die Elektrodenanordnung durch den Instrumentenschaft eingebracht, ohne dass dieser Instrumentenschaft entfernt werden muss. Er verbleibt im Körper. Durch diese Maßnahme sowie durch das einfache und schnelle Aufspannen der Elektrodenanordnung wird die Zeit bis zum Einsatz der flächenhaften Koagulation und die Zeit für die Koagulation selbst erheblich verkürzt.

So kann insbesondere das gesamte Operationsinstrument mit der Resektionsschlinge am Ende einer Operation ausgetauscht werden. Für die flächenhafte Koagulation kann als zweites Arbeitselement dann eine Koagulations-Vorrichtung mit einer entspannten Elektrodenanordnung in den Resektionsschaft eingeführt werden. Ein Aufnahmerohr für eine Endoskop-Optik ist etwas kürzer ausgebildet als ein gewöhnlich benutztes Aufnahmerohr, das sonst bis zum Schaftende reicht. Das Aufnahmerohr kann so weit verkürzt sein, dass es kurz nach einem Bajonettverschluss für die Verriegelung des Stabilisierungsrohres endet. Hierdurch kann die entspannte Elektrodenanordnung auch durch kleine Schaftdurchmesser (z.B.22 Charr. oder kleiner) eingeführt werden. Zunächst wird die Koagulations-Vorrichtung mit der entspannten Elektrodenanordnung durch den Instrumentenschaft eingeführt und mit dem Schaft verriegelt. Dadurch wird erreicht, dass die Elektrodenanordnung aus dem Schaft heräusragt. Anschließend wird die Endoskop-Optik durch das Aufnahmerohr eingeführt und dann die Elektrodenanordnung unter Sicht z.B. in der Prostata-Loge aufgespannt. Das Entfernen der Elektrodenanordnung geschieht in umgekehrter Reihenfolge, d.h. die Elektrodenanordnung wird zunächst entspannt, dann wird die Endoskop-Optik entfernt und anschließend die Koagulations-Vorrichtung mit der entspannten Elektrodenanordnung durch den Instrumentenschaft herausgezogen.

Die als Elektrodenstab ausgebildete Elektrodenanordnung kann eine isolierte Zuleitung, ein Zugkapillarrohr und einen Schlauchüberzug aufweisen und durch ein an einem Aufnahmerohr für die Endoskop-Optik fixiertes Stabilisierungsrohr gleiten. Durch Hin- und Herbewegen z.B. mit dem Daumen kann ähnlich einer Resektionselektrode ein Schlitten aktiv oder passiv bewegt werden. Hierdurch lässt sich die erste und zweite Elektrodengruppe aufspannen, wobei die Umhüllenden der ersten und der zweiten Elektrodengruppe gegeneinander axial verschoben sind. Auch im aufgespannten Zustand haben die Elektroden dieser beiden Elektrodengruppen die gleiche Konfiguration.

In einem ersten Axialbereich liegt die Umhüllende der ersten Elektrodengruppe innerhalb der Umhüllenden der zweiten Elektrodengruppe. In einem daran anschließenden Axialbereich hingegen liegt die Umhüllende der ersten Elektrodengruppe außerhalb der Umhüllenden der zweiten Elektrodengruppe. Die Elektrodenanordnung wird unter Sicht in der Prostata-Loge platziert, um zu vermeiden, dass die Elektroden der Elektrodenanordnung fehlplatziert werden. Hierdurch kann die Elektrodenanordnung optimal in die Prostata-Loge entfaltet werden. Durch die gleichzeitige langsame Zuspülung wird die Bildung von Koageln während der flächenhaften Koagulation verhindert. Die Elektroden liegen fest am Gewebe an und passen sich wegen ihrer flexiblen Ausbildung an Unebenheiten an. Die Koagulation erfolgt in einer Kochsalzlösung zwischen den jeweils benachbarten Elektroden der ersten und zweiten Elektrodengruppe, die im aufgespannten Zustand in gleichem Abstand voneinander gehalten werden.

Ein Vorteil dieser Elektrodenanordnung liegt darin, dass die Elektrodenanordnung im entspannten Zustand durch alle gängigen Resektionsschäfte eingeführt werden kann und unter endoskopischer Sicht z. B. in einer Prostata-Loge platziert und dort aufgespannt werden kann. Mit dem bipolaren Koagulationsstrom zwischen den einzelnen aufgespannten Elektrodenpaaren wird eine großflächige Koagulation durchgeführt, durch die eine venöse Blutung oder kleinere arterielle Blutungen schnell gestoppt wird. Durch eine blutungsfreie Loge am Ende der Operation wird die Katheterverweildauer verkürzt. Es kann auf eine Einlage eines transurethralen Katheters (Harnröhrenkatheters) verzichtet werden.

Im Folgenden wird die Erfindung anhand von in den Figuren 2 bis 10 dargestellten Ausführungsbeispielen beschrieben. Es zeigen:
Figur 2 eine schematische Darstellung einer erfindungsgemäßen Vorrichtung mit wie Meridiane eines Ballons aufgespannter Elektrodenanordnung,
Figur 3 die Ausbildung eines Elektrodensterns der ersten und zweiten Elektroden-Gruppe,
Figur 4 einen Ausschnitt des Zentralbereichs (distales Ende) des Elektrodensterns nach Figur 3,
Figur 5 ein gegenüber liegendes Ende (proximales Ende) einer bandförmigen Elektrode des Elektrodensterns nach Figur 3,
Figur 6 eine räumliche Darstellung der wie Meridiane eines Ballons aufgespannten Elektrodenanordnung,
Figur 7 einen Ausschnitt aus der Darstellung von Figur 6,
Figur 8 eine Anoden- und Kathodenkupplung am distalen Ende der Elektrodenanordnung mit Isolierscheibe,
Figur 9 eine Kupplungsscheibe mit Isolierscheibe am proximalen Ende der Elektrodenanordnung und
Figur 10 einen Elektrodenstab im Schnitt

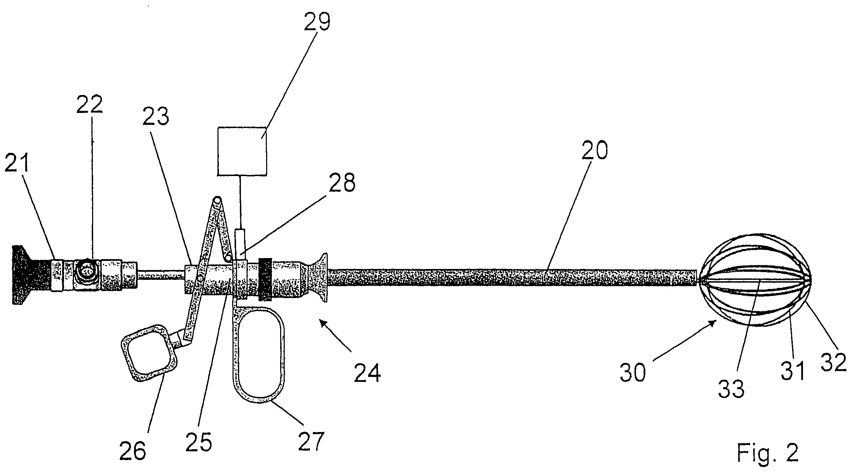

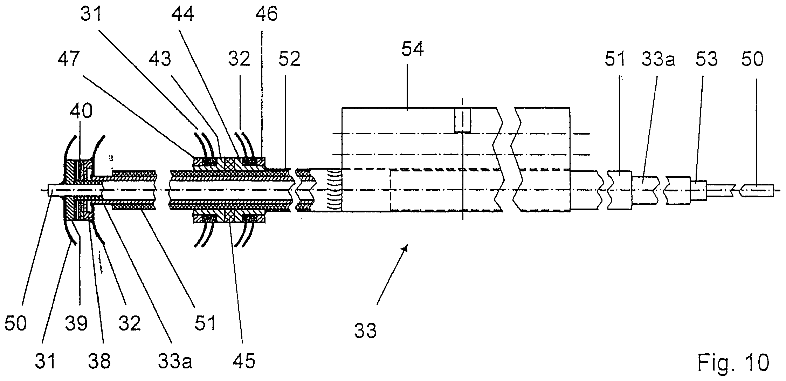

Gemäß Figur 2 enthält ein Resektoskop einen Instrumentenschaft 20 und eine Endoskop-Optik 21 mit einem Anschluss 22 für ein Lichtkabel. Ein Aufnahmerohr 23 für die Optik 21 führt in ein Instrumentengehäuse 24. Ein beweglicher Schlitten 25 ist mit einem durch den Daumen betätigbaren Griff 26 verbunden. Ein Handgriff für den dritten und vierten Finger ist mit 27 bezeichnet. Mit einem Anodenanschluss und Kathodenanschluss 28 ist ein Hochfrequenzgenerator 29 verbunden, der in nicht dargestellter Weise eine hochfrequente Koagulationsspannung an eine Elektrodenanordnung liefert. Diese Elektrodenanordnung umfasst eine erste und eine zweite Elektrodengruppe 31 und 32, die gegeneinander isoliert sind und die jeweils bipolare Elektrodenpaare bilden. Die Elektrodenpaare der ersten und zweiten Elektrodengruppe 31 und 32 sind in Figur 2 durch ein Zugkapillarrohr 33a (Figur 10) eines Elektrodenstabes 33 (Figur 10) wie Meridiane eines Ballons aufgespannt, wobei sich in Umfangsrichtung die Elektroden der einen Elektrodengruppe 31 und der anderen Elektrodengruppe 32 abwechseln.

Mit Bezug auf Figur 2 wird nach einer endoskopischen Operation in nicht dargestellter Weise zunächst die Endoskop-Optik 21 aus dem Instrumentenschaft 20 nach links herausgezogen. Anschließend wird durch den Instrumentenschaft 20 das nicht dargestellte Operationsinstrument entfernt und dann durch den Elektrodenstab 33 mit Elektrodenanordnung 30 ersetzt, der ebenfalls von der linken Seite eingeführt wird, wobei hierbei die Elektrodengruppen 31 und 32 der Elektrodenanordnung 30 entspannt sind und am Zugkapillarrohr 33a des Elektrodenstabes 33 anliegen. Danach wird die Endoskop-Optik 21 wieder eingebracht.

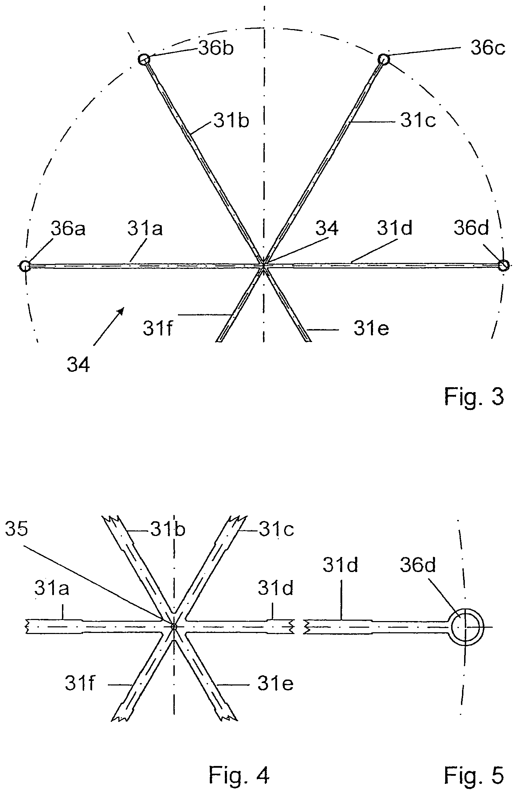

In Figur 3 ist ein Elektrodenstern 34 der Elektrodengruppe 31 im abgewickelten (nicht eingebauten) Zustand dargestellt. Die einzelnen Elektroden bzw. Elektrodenarme 31a, 31b, 31c, 31d, 31e und 31f sind bandförmig und flexibel und elektrisch leitend. Bei dem dargestellten Ausführungsbeispiel sind sechs Elektroden 31a - 31f sternförmig angeordnet und an einem Zentralbereich einstückig miteinander verbunden. Der Zentralbereich weist gemäß Figur 4 eine zentrale Öffnung 35 für eine elektrische Zuleitung 50 (Figur 10) auf. Die in Figur 3 äußeren Enden 36a - 36f des Elektrodensterns 34, die in Figur 5 vergrößert dargestellt sind, weisen Öffnungen auf, um einen Durchtritt des Zugkapillarrohrs 33a des Elektrodenstabes 33 zu ermöglichen. Die einzelnen Elektrodenarme, die elastisch verformbar sind, sind zu ihren jeweiligen Enden hin schmäler ausgebildet, wie insbesondere aus den Figuren 4 und 5 ersichtlich ist, d.h. sie weisen Endabschnitte mit verringerter Breite auf. Hierdurch wird der Koagulationseffekt in diesem Bereich verringert.

Alternativ hierzu oder auch zur Optimierung der Verringerung des Koagulationseffektes können die jeweiligen Endbereiche gemäß einer weiteren Ausführung mit einer Isolierung versehen sein, die sich beispielsweise über etwa die letzten 5 - 10 mm erstreckt. Hierdurch wird vermieden, dass z.B. der nicht dargestellte apikale (untere) Bereich der Prostata mit dem darunter liegenden Schließmuskel sowie der in die Blase hineinreichende Teil des Gewebes bei einer Koagulation zu stark erhitzt wird.

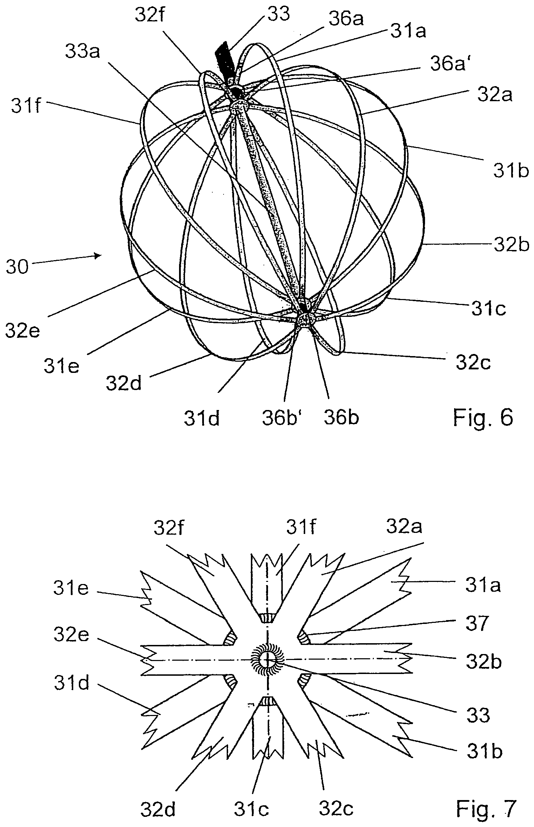

In Figur 6 ist die Elektrodenanordnung 30 im aufgespannten Zustand dargestellt. Die Elektroden 31a - 31f bilden die erste Elektrodengruppe 31, während Elektroden 32a - 32f die zweite Elektrodengruppe32 bilden. Das Kapillarzugrohr 33a des Elektrodenstabes 33 ermöglicht die Axialverschiebung der distalen Enden der ersten und zweiten Elektrodengruppe 31 und 32 in die zum Ballon aufgespannte Stellung. Sämtliche äußeren Endbereiche 36a - 36f bzw. 36a' - 36f der einzelnen Elektroden 31a - 31f und 32a - 32f sind jeweils mit einer Isolierschicht versehen.

Wie insbesondere die Figuren 6 und 7 zeigen, ist die erste Elektroden-Gruppe 31 gegenüber der zweiten Elektroden-Gruppe 32 axial so gedreht angeordnet, dass die bandförmigen Elektrodenarme 31a - 31f der ersten Elektroden-Gruppe 31 jeweils zwischen den bandförmigen Elektrodenarmen 32a - 32f der zweiten Elektroden-Gruppe 32 verlaufen und jeweils bipolare Elektroden-Paare bilden.

In einem ersten (distalen) Axialbereich liegt die Umhüllende der ersten Elektrodengruppe 31 innerhalb der Umhüllenden der zweiten Elektrodengruppe 32. In einem sich daran anschließenden (proximalen) Axialbereich hingegen liegt die Umhüllende der ersten Elektrodengruppe 31 außerhalb der Umhüllenden der zweiten Elektrodengruppe 32, d.h. die beiden Umhüllenden schneiden sich (vgl. auch Figur 2).

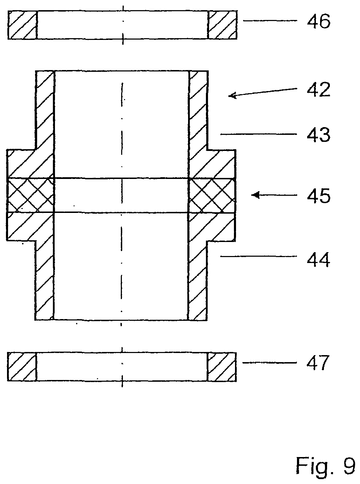

In Figur 7 ist ein Ausschnitt des proximalen Endbereichs der beiden Elektrodengruppen 31 und 32 dargestellt. Die Elektroden 31a - 31f der ersten Elektrodengruppe 31 sind gegenüber den Elektroden 32a - 32f in Umfangsrichtung um die Mittelachse gedreht angeordnet, sodass die Elektroden 31a, 32a - 31f, 32f jeweils Elektrodenpaare bilden. Mit 37 ist ein in Figur 9 vergrößert dargestelltes Kupplungselement bezeichnet.

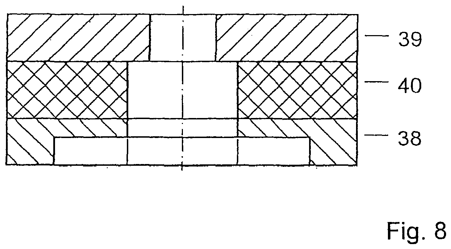

In Figur 8 ist eine Anodenkupplung 38 sowie eine Kathodenkupplung 39 für die distalen Enden der Elektroden der ersten und zweiten Elektrodengruppe 31 und 32 dargestellt. Zwischen der Anodenkupplung und der Kathodenkupplung befindet sich eine die beiden Kupplungen isolierende Scheibe 40 (vgl. auch Figur 10).

In Figur 9 ist eine Kupplungshülse 42 dargestellt, die die zwei gegeneinander isolierte Kupplungshülsenteile 43 und 44 aufweist sowie eine dazwischen befindliche Isolation 45. Diese Kupplung ist auf beiden Seiten mit Sicherungsscheiben 46 und 47 versehen. Im eingebauten Zustand kann die Kupplungshülse 42 axial über dem Elektrodenstab 33 gleiten, sodass ein Schiebemechanismus erreicht werden kann.

Dieser Schiebemechanismus wird durch einen Handgriff betätigt, so wie dies in Figur 2 zu sehen ist, .z. B. durch Daumenbewegung. Eine nicht dargestellte Feststellschraube bestehend aus einer Zylinderschraube, einer Klemmschraube und einer Rändelschraube arretiert das Zugkapillarrohr 33a des Elektrodenstabes 33. Die Teile des Elektrodenstabes 33 werden mit Hilfe eines nicht näher dargestellten Schlittens 25 auf einem Aufnahmerohr 23 der Endoskop-Optik 21 bewegt, sodass dadurch die Elektrodengruppen 31 und 32 wie die Meridiane eines Ballons aufgespannt und wieder entspannt werden können. Die Feststellschraube wirkt als Klemmelement wie ein beweglicher Schlitten, der die Ankopplung der Elektrode bewerkstelligt.

Figur 10 zeigt den Elektrodenstab 33 im Detail. Er weist einen konzentrisch bzw. koaxial angeordneten Aufbau auf, wobei im Inneren die elektrische Zuleitung 50 bzw. das Elektrodenkabel angeordnet ist, das mit einer Isolierung 53 isoliert ist, z.B. mit Teflon. Über dieser isolierten Leitung verläuft das Zugkapillarrohr 33a, das bis zum vorderen Ende der Elektroden 31a - 31f und 32a - 32f der beiden Elektrodengruppen 31 und 32 verläuft und dort mit der Kupplungseinheit von Figur 8 verbunden ist.

Über dem Zugkapillarrohr 33a befindet sich ein Isolationsschlauch 51, der ebenfalls bis zum vorderen Ende verläuft. Die gesamte Anordnung ist durch ein Stabilisierungsrohr 52 geführt, an dessen distalem Ende die Kupplungshülse 42 (Figur 9) befestigt ist.

Die isolierte elektrische Zuleitung 50 gleitet im Inneren des Stabilisierungsrohres 52, das geschlossen oder auch teilweise längs offen ausgebildet sein kann. Der Schiebemechanismus ermöglicht das Öffnen oder Schießen bzw. Aufspannen und Entspannen der beiden Elektrodengruppen 31 und 32 durch Verschieben des Zugkapillarrohres 33a relativ zum Stabilisierungsrohr 52. Die Bewegung des Elektrodenstabes 33 erfolgt so, dass das Zugkapillarrohr 33a an einem beweglichen Schlitten 25 fixiert ist, der mit dem Daumengriff des Arbeitselements bewegt wird. Das Zugkapillarrohr 33a, das mit dem Isolationsschlauch 51 versehen ist, gleitet somit im Stabilisierungsrohr 52, das am Aufnahmerohr 8 für die Optik 2 fixiert ist. Der Elektrodenstab 33 ist bevorzugt unterhalb des Aufnahmerohres 8 für die Optik fixiert.

Das Stabilisierungsrohr 52 weist noch eine Arretierung z.B. in Form eines Bajonettverschlusses 54 auf, so dass die gesamte Anordnung fest am Arbeitselement verbleibt. Mit diesem Bajonettverschluss wird das Aufnahmerohr für die Optik im Endoskop mit dem Stabilisierungsrohr 52 des Elektrodenstabes 33 verbunden.

Unmittelbar hinter der Arretierung für das Zugkapillarrohr 33a des Elektrodenstabes 33 am Schlitten befindet sich noch eine elektrische Steckverbindung für das Elektrodenkabel 50.

Die Elektrodenanordnung wird unter Sicht in der Prostata-Loge platziert, um zu vermeiden, dass die Elektroden der Elektrodenanordnung fehlplatziert werden. Hierdurch kann die Elektrodenanordnung optimal in die Prostata-Loge entfaltet werden. Durch die gleichzeitige langsame Zuspülung wird die Bildung von Koageln während der flächenhaften Koagulation verhindert. Die Elektroden liegen fest am Gewebe an und passen sich wegen ihrer flexiblen bzw. elastischen Ausbildung an Unebenheiten an.

## Patentansprüche

1. Elektrodenanordnung zur Koagulation von Körpergewebe und/oder von Körpergefäßen, umfassend
eine erste Elektroden-Gruppe (31) und eine zweite Elektroden-Gruppe (32) die gegeneinander isoliert sind und die jeweils bandförmige Elektroden (31a - 31f; 32a - 32f) mit proximalen und distalen Enden aufweisen, wobei die proximalen Enden relativ zu den distalen Enden entlang einer Achse axial verschiebbar sind, so dass die Elektroden-der ersten und der zweiten Elektroden-Gruppe (31a - 31f; 32a - 32f) von einer entspannten Stellung in eine aufgespannte Stellung wie Meridiane eines Ballons aufspannbar sind,
**dadurch gekennzeichnet, dass** die erste Elektroden-Gruppe gegenüber der zweiten Elektroden-Gruppe axial versetzt angeordnet ist, sodass sich die Umhüllende der ersten Elektroden-Gruppe (31) und die Umhüllende der zweiten Elektroden-Gruppe (32) derart schneiden, dass im aufgespannten Zustand der Elektroden die Umhüllende der ersten Elektroden-Gruppe (31) in einem ersten Axialbereich außerhalb und in einem anschließenden zweiten Axialbereich innerhalb der Umhüllenden der zweiten Elektroden-Gruppe (32) liegt.

2. Elektrodenanordnung nach Anspruch 1,
**dadurch gekennzeichnet,**
**dass** die erste Elektroden-Gruppe (31) gegenüber der zweiten Elektroden-Gruppe (32) um die Achse so gedreht angeordnet ist, dass die Elektroden (31a - 31f) der ersten Elektroden-Gruppe (31) in Umfangsrichtung gesehen jeweils zwischen den Elektroden (32a - 32f) der zweiten Elektroden-Gruppe (32) verlaufen.

3. Elektrodenanordnung nach Anspruch 1 oder 2,
**dadurch gekennzeichnet,**
**dass** die Elektroden der ersten und zweiten Elektroden-Gruppe (31, 32) in ihren jeweiligen Endbereichen mit einer Isolation (36a, 36b, 36a', 36b') versehen sind, die sich insbesondere über etwa die letzten 3 - 15 mm, bevorzugt über etwa die letzten 5 - 10 mm erstreckt.

4. Elektrodenanordnung nach zumindest einem der vorstehenden Ansprüche,
**dadurch gekennzeichnet,**
die distalen Enden jeweils von einem ersten zentralen Elektroden-Aufnahmeelement (38, 39, 40) gehalten sind, und
dass ein relativ zu dem ersten zentralen Elektroden-Aufnahmeelement (38, 39 40) axial verschiebbares zweites Elektroden-Aufnahmeelement (42) zur Aufnahme der proximalen Enden vorgesehen ist.

5. Elektrodenanordnung nach zumindest einem der vorstehenden Ansprüche,
**dadurch gekennzeichnet,**
**dass** sich die Breite zumindest einiger Elektroden zu ihrem jeweiligen Ende hin verringert.

6. Elektrodenanordnung nach zumindest einem der vorstehenden Ansprüche,
**dadurch gekennzeichnet,**
**dass** diese einen Elektrodenstab (33) aufweist, der ein inneres isoliertes Kabel (50) umfasst, das durch ein Zugkapillarrohr (33a) geführt ist, wobei das Zugkapillarrohr (33a) außen mit einem Isolierschlauch (51) überzogen ist, der in einem Stabilisierungsrohr (52) geführt ist.

7. Elektrodenanordnung nach Anspruch 6,
**dadurch gekennzeichnet,**
**dass** das Stabilisierungsrohr (52) des Elektrodenstabes (33) über dem Schlauch (51) durch einen Handgriff an einem Arbeitselement eines Endoskops bewegbar ist, und
**dass** das Zugkapillarrohr (33a) mit dem Schlauch (51) und dem isolierten Kabel (50) durch das Stabilisierungsrohr (52) bewegbar sind.

8. Elektrodenanordnung nach Anspruch 6,
**dadurch gekennzeichnet,**
**dass** das Stabilisierungsrohr (52) mit einem Verschluss (54) an einem Aufnahmerohr für eine Optik eines Endoskops fixierbar ist, und
**dass** auf dem Aufnahmerohr ein Schlitten gleitend vorgesehen ist, der das Zugkapillarrohr (33a) mittels eines Klemmelementes fixiert, so dass durch Bewegung des Schlittens der Elektrodenstab (33) gegenüber dem Stabilisierungsrohr (52) zum Bewegen der Elektroden in die aufgespannte Stellung bewegbar ist.

9. Elektrodenanordnung nach zumindest einem der vorstehenden Ansprüche,
**dadurch gekennzeichnet,**
**dass** die Elektroden der ersten und zweiten Elektroden-Gruppe (31, 32) mit ihren distalen Enden jeweils einen ersten und zweiten Zentralbereich bilden, der jeweils in einem zugehörigen ersten zentralen Elektroden-Aufnahmeelement (38, 39, 40) gehalten ist, und
**dass** die proximalen Enden der Elektroden der ersten und zweiten Elektroden-Gruppe (31, 32) in einer ringförmigen Aufnahme (42) gehalten sind.

10. Elektrodenanordnung nach zumindest einem der vorstehenden Ansprüche,
**dadurch gekennzeichnet,**
**dass** die distalen Enden der Elektroden einer Elektroden-Gruppe (31) mit einer Kathoden-Kupplung (39) und die distalen Enden der Elektroden der anderen Elektroden-Gruppe (32) mit einer AnodenKupplung (38) verbunden sind.

11. Elektrodenanordnung nach zumindest einem der vorstehenden Ansprüche,
**dadurch gekennzeichnet,**
**dass** die proximalen Enden der Elektroden einer Elektroden-Gruppe (31) mit einer Kathoden-Kupplungshülse (44) und die proximalen Enden der Elektroden der anderen Elektroden-Gruppe (32) mit einer gegenüber der Kathoden-Kupplungshülse (44) isolierten Anoden-Kupplungshülse (43) verbunden sind.

12. Elektrodenanordnung nach Anspruch 11,
**dadurch gekennzeichnet,**
**dass** die Kathoden-Kupplungshülse (44) und die Anoden-Kupplungshülse (43) durch Sicherungsscheiben (46, 47) gesichert sind.

13. Elektrodenanordnung nach zumindest einem der vorstehenden Ansprüche,
**dadurch gekennzeichnet,**
**dass** diese mit einem Endoskop, insbesondere mit einem Resektoskop, verbunden ist, derart, dass die Elektroden der ersten und zweiten Elektroden-Gruppe (31,32) in der entspannten Stellung in einen Instrumentenschaft (20) des Endoskops einschiebbar und dann mit diesem verriegelbar sind.

## Claims

1. An electrode arrangement for coagulating body tissues and/or body vessels, comprising
a first electrode group (31) and a second electrode group (32) which are insulated with respect to one another and which each have ribbon-shaped electrodes (31a - 31f; 32a - 32f) having proximal and distal ends, wherein the proximal ends are axially displaceable along an axis relative to the distal ends so that the electrodes of the first and second electrode groups (31a - 31f; 32a - 32f) can be expanded from a relaxed position into an expanded position like meridians of a balloon,
**characterized in that**
the first electrode group is arranged axially offset with respect to the second electrode group so that the envelope of the first electrode group (31) and the envelope of the second electrode group (32) intersect such that, in the expanded state of the electrodes, the envelope of the first electrode group (31) lies outside the envelope of the second electrode group (32) in a first axial region and inside the envelope of the second electrode group (32) in an adjoining second axial region.

2. An electrode arrangement in accordance with claim 1,
**characterized in that**
the first electrode group (31) is arranged rotated about the axis with respect to the second electrode group (32) in a manner such that the electrodes (31a - 31f) of the first electrode group (31) extend in each case, viewed in the peripheral direction, between the electrodes (32a - 32f) of the second electrode group (32).

3. An electrode arrangement in accordance with claim 1 or claim 2,
**characterized in that**
the electrodes of the first and second electrode groups (31, 32) are provided in their respective end regions with an insulation (36a, 36b, 36a', 36b'), which in particular extends over approximately the last 3 - 15 mm, preferably over approximately the last 5 - 10 mm.

4. An electrode arrangement in accordance with at least one of the preceding claims,
**characterized in that**
the distal ends are each held by a first central electrode receiving element (38, 39, 40); and
**in that** a second electrode receiving element (42) axially displaceable relative to the first central electrode receiving element (38, 39, 40) is provided for receiving the proximal ends.

5. An electrode arrangement in accordance with at least one of the preceding claims,
**characterized in that**
the width of at least some electrodes reduces toward their respective ends.

6. An electrode arrangement in accordance with at least one of the preceding claims,
**characterized in that**
it has an electrode rod (33) which comprises an inner insulated cable (50) which is led through a capillary draw tube (33a), with the capillary draw tube (33a) being sheathed outwardly by an insulating hose (51) which is led in a stabilization tube (52).

7. An electrode arrangement in accordance with claim 6,
**characterized in that**
the stabilization tube (52) of the electrode rod (33) is movable over the hose (51) by a movement of the hand at a working element of an endoscope; and
**in that** the capillary draw tube (33a) with the hose (51) and the insulated cable (50) are movable through the stabilization tube (52).

8. An electrode arrangement in accordance with claim 6,
**characterized in that**
the stabilization tube (52) can be fixed using a closure (54) at a receiving tube for an optics of an endoscope; and
**in that** a carriage is provided in a sliding manner on the receiving tube and fixes the capillary draw tube (33a) by means of a clamping element so that, by moving the carriage, the electrode rod (33) is movable with respect to the stabilization tube (52) for moving the electrodes into the expanded position.

9. An electrode arrangement in accordance with at least one of the preceding claims,
**characterized in that**
the electrodes of the first and second electrode groups (31, 32) each form a first and second central region with their distal ends which is each held in an associated first central electrode receiving element (38, 39, 40); and
**in that** the proximal ends of the electrodes of the first and second electrode groups (31, 32) are held in a ring-shaped receiver (42).

10. An electrode arrangement in accordance with at least one of the preceding claims,
**characterized in that**
the distal ends of the electrodes of an electrode group (31) are connected to a cathode coupling (39) and the distal ends of the electrodes of the other electrode group (32) are connected to an anode coupling (38).

11. An electrode arrangement in accordance with at least one of the preceding claims,
**characterized in that**
the proximal ends of the electrodes of an electrode group (31) are connected to a cathode coupling sleeve (44) and the proximal ends of the electrodes of the other electrode group (32) are connected to an anode coupling sleeve (43) insulated with respect to the cathode coupling sleeve (44).

12. An electrode arrangement in accordance with claim 11,
**characterized in that**
the cathode coupling sleeve (44) and the anode coupling sleeve (43) are secured by securing plates (46, 47).

13. An electrode arrangement in accordance with at least one of the preceding claims,
**characterized in that**
it is connected to an endoscope, in particular to a rectoscope, such that the electrodes of the first and second electrode groups (31, 32) can be pushed into an instrument shaft (20) of the endoscope in the relaxed position and can then be latched thereto.

## Revendications

1. Ensemble d'électrodes pour la coagulation de tissu corporel et/ou de vaisseaux corporels, comportant
un premier groupe d'électrodes (31) et un second groupe d'électrodes (32) qui sont isolés l'un par rapport à l'autre et qui comprennent chacun des électrodes en forme de bande (31a - 31f ; 32a - 32f) ayant des extrémités proximales et distales, les extrémités proximales étant mobiles en translation par rapport aux extrémités distales axialement le long d'un axe, de sorte que les électrodes des premier et second groupes d'électrodes (31a - 31f ; 32a - 32f) sont susceptibles d'être tendues depuis une position détendue jusque dans une position tendue à la manière des méridiens d'un ballon,
**caractérisé en ce que**
le premier groupe d'électrodes est agencé axialement en décalage par rapport au second groupe d'électrodes, de sorte que l'enveloppante du premier groupe d'électrodes (31) et l'enveloppante du second groupe d'électrodes (32) se coupent de telle sorte que dans l'état tendu des électrodes, l'enveloppante du premier groupe d'électrodes (31) se situe dans une première zone axiale à l'extérieur de l'enveloppante du second groupe d'électrodes (32) et dans une seconde zone axiale suivante elle se situe à l'intérieur de celle-ci.

2. Ensemble d'électrodes selon la revendication 1,
**caractérisé en ce que**
le premier groupe d'électrodes (31) est agencé de façon tournée par rapport au second groupe d'électrodes (32) autour de l'axe, de telle sorte que les électrodes (31a - 31f) du premier groupe d'électrodes (31) s'étendent chacune entre les électrodes (32a - 32f) du second groupe d'électrodes (32), vues en direction périphérique.

3. Ensemble d'électrodes selon la revendication 1 ou 2,
**caractérisé en ce que**
dans leurs zones d'extrémité respectives, les électrodes des premier et second groupes d'électrodes (31, 32) sont pourvues d'une isolation (36a, 36b, 36a', 36b') qui s'étend en particulier approximativement sur les derniers 3 - 15 mm, de préférence approximativement sur les derniers 5 - 10 mm.

4. Ensemble d'électrodes selon l'une au moins des revendications précédentes,
**caractérisé en ce que**
les extrémités distales sont retenues chacune par un premier élément de réception d'électrode (38, 39, 40) central, et
**en ce qu'**un second élément de réception d'électrode (42) axialement mobile en translation par rapport au premier élément de réception d'électrode (38, 39, 40) central est prévu pour recevoir les extrémités proximales.

5. Ensemble d'électrodes selon l'une au moins des revendications précédentes,
**caractérisé en ce que**
la largeur d'au moins quelques-unes des électrodes diminue vers leur extrémité respective.

6. Ensemble d'électrodes selon l'une au moins des revendications précédentes,
**caractérisé en ce que**
celui-ci comprend une tige d'électrode (33) qui présente un câble intérieur isolé (50) qui est mené à travers un tube capillaire de traction (33a), le tube capillaire de traction (33a) étant revêtu à l'extérieur d'une gaine isolante (51) qui est menée dans un tube de stabilisation (52).

7. Ensemble d'électrodes selon la revendication 6,
**caractérisé en ce que**
le tube de stabilisation (52) de la tige d'électrode (33) est mobile par-dessus la gaine (51) par une manette sur un élément de travail d'un endoscope, et le tube capillaire de traction (33a) est mobile avec la gaine (51) et avec le câble isolé (50) à travers le tube de stabilisation (52).

8. Ensemble d'électrodes selon la revendication 6,
**caractérisé en ce que**
le tube de stabilisation (52) peut être fixé par un obturateur (54) sur un tube de réception pour une optique d'un endoscope, et
**en ce qu'**un chariot est prévu en coulissement sur le tube de réception, qui fixe le tube capillaire de traction (33a) au moyen d'un élément de coincement, de sorte que par le mouvement du chariot la tige d'électrode (33) est mobile par rapport au tube de stabilisation (52) pour déplacer les électrodes jusque dans la position tendue.

9. Ensemble d'électrodes selon l'une au moins des revendications précédentes,
**caractérisé en ce que**
les électrodes des premier et second groupes d'électrodes (31, 32) forment de par leurs extrémités distales chacune une première et une seconde zone centrale qui est retenue dans un premier élément de réception d'électrode (38, 39, 40) central associé respectif, et
**en ce que** les extrémités proximales des électrodes du premier et du second groupe d'électrodes (31, 32) sont retenues dans un logement annulaire (42).

10. Ensemble d'électrodes selon l'une au moins des revendications précédentes,
**caractérisé en ce que**
les extrémités distales des électrodes d'un groupe d'électrodes (31) sont reliées à un couplage de cathode (39) et les extrémités distales des électrodes de l'autre groupe d'électrodes (32) sont reliées à un couplage d'anode (38).

11. Ensemble d'électrodes selon l'une au moins des revendications précédentes,
**caractérisé en ce que**
les extrémités proximales des électrodes d'un groupe d'électrodes (31) sont reliées à une douille de couplage de cathode (44), et les extrémités proximales des électrodes de l'autre groupe d'électrodes (32) sont reliées à une douille de couplage d'anode (43) isolée par rapport à la douille de couplage de cathode (44).

12. Ensemble d'électrodes selon la revendication 11,
**caractérisé en ce que**
la douille de couplage de cathode (44) et la douille de couplage d'anode (43) sont bloquées par des rondelles de blocage (46, 47).

13. Ensemble d'électrodes selon l'une au moins des revendications précédentes,
**caractérisé en ce que**
celui-ci est relié à un endoscope, en particulier à un résectoscope, de telle sorte que dans l'état détendu, les électrodes des premier et second groupes d'électrodes (31, 32) sont susceptibles d'être introduites dans une tige d'instrument (20) de l'endoscope et sont ensuite verrouillables avec celle-ci.
